# EUROPÄISCHE PATENTSCHRIFT

(11) **EP 3 527 999 B1**
(45) Veröffentlichungstag und Bekanntmachung des Hinweises auf die Patenterteilung: **27.03.2024**
(21) Anmeldenummer: 18157062.3
(22) Anmeldetag: 16.02.2018
(51) Int. Cl.: G01R 33/34, G01R 33/422, G01R 33/36

(54) **SENDEANTENNE FÜR EINE MAGNETRESONANZEINRICHTUNG**
TRANSMITTING ANTENNA FOR A MAGNETIC RESONANCE DEVICE
ANTENNE ÉMETTRICE POUR UN DISPOSITIF DE RÉSONANCE MAGNÉTIQUE

(43) Veröffentlichungstag der Anmeldung: 21.08.2019
(73) Patentinhaber: Siemens Healthineers AG, 91301 Forchheim (DE)
(72) Erfinder: Nistler, Jürgen, 91056 Erlangen (DE)
(74) Vertreter: Siemens Healthineers Patent Attorneys

(56) Entgegenhaltungen:
- DE-A1-102008 006 117
- US-A1- 2012 025 833
- US-A1- 2012 262 173
- NICOLA DE ZANCHE ET AL: "Algebraic method to synthesize specified modal currents in ladder resonators: Application to noncircular birdcage coils : Ladder Resonator Synthesis", MAGNETIC RESONANCE IN MEDICINE., Bd. 74, Nr. 5, 13. November 2014 (2014-11-13), Seiten 1470-1481, XP55493150, US ISSN: 0740-3194, DOI: 10.1002/mrm.25503

## Beschreibung

Die Erfindung betrifft eine Sendeantenne für eine Magnetresonanzeinrichtung mit mehreren in eine Umfangsrichtung voneinander beabstandet um eine Mittelgerade herum angeordneten, parallel zu der Mittelgerade verlaufenden Antennenleitern und einem parallel zu der Mittelgerade verlaufenden, die Antennenleiter umgreifenden Abschirmelement, wobei für wenigstens ein Paar der Antennenleiter der radiale Abstand zwischen einem ersten Antennenleiter des Paars und dem Abschirmelement kleiner ist als der radiale Abstand zwischen einem zweiten Antennenleiter des Paars und dem Abschirmelement, wobei die Breite des ersten Antennenleiters in Umfangsrichtung kleiner als die Breite des zweiten Antennenleiters in Umfangsrichtung ist und/oder wobei die axialen Enden des ersten Antennenleiters über eine größere Kapazität miteinander gekoppelt sind als die axialen Enden des zweiten Antennenleiters, wobei die Antennenleiter an ihren Enden Endringabschnitte aufweisen, wobei in Umfangsrichtung der Sendeantenne benachbarte Endringabschnitte kapazitiv oder leitend gekoppelt sind. Daneben betrifft die Erfindung eine Magnetresonanzeinrichtung und ein Verfahren zur Herstellung einer Sendeantenne.

Die Magnetresonanztomographie ist, insbesondere in der medizinischen Bildgebung, mittlerweile ein häufig genutztes Verfahren zur dreidimensionalen Bildgebung. Hierbei werden Kernspins in einem Objekt, beispielsweise in einem Patienten, durch hochfrequente elektromagnetische Wellen angeregt und beim Abklingen dieser Anregung abgestrahlte elektromagnetische Wellen werden empfangen und zur Bildgebung ausgewertet. Eine Möglichkeit entsprechende hochfrequente Wellen einzustrahlen sind zylinderförmige Antennen, die ein Untersuchungsobjekt oder Teile eines Untersuchungsobjekts in ihrem Innenraum aufnehmen. Hierbei werden mehrere parallele Antennenleiter genutzt, die insbesondere entlang der Mantelfläche eines Zylinders angeordnet werden. Um eine Störung weiterer Einrichtungen außerhalb des Messbereichs zu vermeiden, sind diese Antennenleiter von einem Abschirmelement, beispielsweise einem zylinderförmigen Schild, umgeben. Solchen Sendeantennen werden üblicherweise über beabstandete Anschlusspunkte identische Anregungssignale mit einem Phasenversatz von 90° zugeführt, um ein zirkular polarisiertes B1-Feld zur Anregung von Kernspins bereit zu stellen. Durch eine Modifikation der Anregung oder der Antennengeometrie kann gemäß der Druckschrift DE 10 2008 006 117 A1 auch eine elliptische Polarisierung der abgestrahlten Welle erreicht werden.

Aus dem Artikel Nicola De Zanche et al: "Algebraic method to synthesize specified modal currents in ladder resonators: Application to noncircular birdcage coils", MAGNETIC RESONANCE IN MEDICINE, Bd. 74, Nr. 5, 13. November 2014 (2014-11-13), Seiten 1470-1481, XP55493150, US, ISSN: 0740-3194, DOI: 10.1002/mrm.25503, ist es bekannt, eine elliptische Käfigantenne in einem kreisförmigen Hochfrequenzschild anzuordnen. Hierbei werden optimale Kapazitäten für die Käfigantenne in Abhängigkeit der Form der Abschirmung der Käfigantenne berechnet.

Die Druckschrift US 2012/0 262 173 A1 offenbart eine Hochfrequenzspule für eine Magnetresonanzbildgebung. Hierbei können die Abstände der genutzten Hochfrequenzspulen von einer Hochfrequenzabschirmung variieren. Zur Abstimmung der Resonanzfrequenz der gebildeten Leiterschleifen bzw. Halbschleifen können in diese geschaltete Kapazitäten angepasst werden.

Die Druckschrift US 2012/0 025 833 A1 offenbart eine Magnetresonanzeinrichtung mit einer um eine Ausnehmung angeordneten Hochfrequenzspule und einer Hochfrequenzabschirmung. Um bei Nutzung einer asymmetrischen Hochfrequenzspule ein möglichst homogenes magnetisches Feld zu erreichen, können die Impedanzen in der Hochfrequenzspule angepasst werden.

Um die Verfügbarkeit von Magnetresonanztomographen zu verbessern, ist es vorteilhaft, wenn diese möglichst günstig und kleinbauend realisiert werden. Ein Ansatz dies zu erreichen ist die Reduzierung der Höhe des Innenraums einer im Magnetresonanztomographen genutzten Ganzkörper-Antenne. Um dennoch eine ausreichende Breite zur Aufnahme von Untersuchungsobjekten, insbesondere von Patienten, zu ermöglichen, kann es hierbei erforderlich sein, bei der Anordnung der Antennenleiter beziehungsweise bei der Form eines genutzten Abschirmelements von der üblichen Zylinderflächenform abzuweichen. Problematisch hierbei ist bislang, dass die Homogenität des B1-Feldes und somit typischerweise auch die erreichbare Messqualität mit einer zunehmenden Abweichung von einer Zylindermantelflächenform abnimmt. Zudem führen solche Abweichungen auch zu einer ungleichmäßigen Verteilung des Stroms auf die verschiedenen Antennenleiter, womit einerseits eine höhere Bauteilbelastung resultiert und andererseits spezifische Absorptionsraten für Abschnitte des Untersuchungsobjekts, die nahe an den hochbestromten Antennenleitern liegen, erhöht werden.

Der Erfindung liegt somit die Aufgabe zugrunde, die genannten Nachteile bei einer kosten- beziehungsweise bauraumoptimierten Bauweise einer Sendeantenne zumindest teilweise zu kompensieren.

Die Aufgabe wird erfindungsgemäß dadurch gelöst, dass bei einer Sendeantenne der eingangs genannten Art die Ausdehnungen der Endringabschnitte der Antennenleiter des Paares in Axialrichtung unterschiedlich voneinander sind .

Im Rahmen der Erfindung wurde erkannt, dass die eingangs erläuterten Nachteile bei einer Abweichung der Anordnung der Antennenleiter beziehungsweise der Form des Abschirmelements von einer idealen Zylinderform größtenteils dadurch verursacht werden, dass aufgrund unterschiedlicher radialer Abstände der Antennenleiter von dem Abschirmelement die Induktivität der einzelnen Antennenleiter und somit der Wechselstromwiderstand für die genutzten Hochfrequenzsignale zwischen den Antennenleitern variiert. Bei einer gleichen Ausgestaltung der Antennenleiter steigt die Induktivität des einzelnen Antennenleiters und somit dessen Wechselstromwiderstand mit zunehmendem radialen Abstand von dem Abschirmelement. Hieraus resultieren relativ große Ströme für Antennenleiter, deren radialer Abstand zum Abschirmelement relativ klein ist und umgekehrt.

Erfindungsgemäß wird nun vorgeschlagen, von der Gleichgestaltung der Antennenleiter abzuweichen, wobei die Breite der Antennenleiter variiert wird. Dadurch, dass die Ausdehnungen der Endringabschnitte der Antennenleiter des Paares in Axialrichtung der Sendeantenne unterschiedlich voneinander sind, kann die obig beschriebene Variation des Wechselstromwiderstands und somit des Stromflusses zwischen den Antennenleitern zumindest teilweise kompensiert werden. Hierdurch kann, wenn ein zirkular polarisiertes B1-Feld abgestrahlt werden soll, dessen Homogenität verbessert werden. Zudem können aufgrund der gleichmäßigen Stromverteilung auf die Antennenleiter die maximale Bauteilbelastung der Sendeantenne und die lokalen Maxima der spezifischen Absorptionsrate reduziert oder vermieden werden.

Vorteilhafte Breiten können abgeschätzt werden, indem theoretische Formeln für die Induktivität eines geraden Drahtes in der Nähe einer leitenden Wand betrachtet werden. Wird das Abschirmelement beispielsweise als ideal leitende Wand angenähert und die Antennenleiter als gerade Drähte, so ist die Induktivität proportional zum Logarithmus des Koeffizienten aus dem doppelten Abstand des jeweiligen Antennenleiters von dem Abschirmelement und dem Drahtdurchmesser. In einer ersten Näherung könnte somit die Breite der Antennenleiter jeweils so gewählt werden, dass der Koeffizient aus Abstand und Breite näherungsweise konstant ist. Bessere Näherungen können durch komplexere Modellrechnungen und insbesondere durch die Nutzung von dreidimensionalen Feldsimulationen ermittelt werden.

Die Nutzung von zunehmend kleineren Kapazitäten mit wachsendem Abstand kann ergänzend hierzu genutzt werden, um die Wechselstromwiderstände der Antennenleiter aneinander anzugleichen. Hierbei kann der Antennenleiter als Serienschaltung einer parasitären Induktivität und der zusätzlich in den Antennenleiter geschalteten Kapazität betrachtet werden. Dies entspricht einem Reihenschwingkreis, der einen frequenzabhängigen Wechselstromwiderstand aufweist, wobei der Wechselstromwiderstand ausgehend von einem leitenden Draht, also einer näherungsweise unendlichen Kapazität, mit sinkender Kapazität zunächst sinkt, bis eine Kapazitätsschwelle, die dem Resonanzfall des Reinschwingkreises bei der momentanen Frequenz entsprechen, unterschritten wird. Werden keine allzu kleinen Kapazitäten genutzt, so führt somit die Nutzung einer kleineren Kapazität mit zunehmendem Abstand des jeweiligen Antennenleiters von dem Abschirmelement zu einer zumindest teilweisen Kompensation der durch die Abstandsänderung resultierenden Änderung des Wechselstromwiderstandes. Die genutzten Kapazitäten können wiederum mit entsprechenden Modellen und Näherungen der Sendeantenne berechnet werden oder beispielsweise im Rahmen der Lösung eines Optimierungsproblems, das eine dreidimensionale Feldsimulation nutzt, ermittelt werden.

Die beschriebenen Ansätze zur Kompensation eines Wechselstromwiderstandes, also die Variation der Breite des Antennenleiters beziehungsweise seiner Kapazität, können ausschließlich für ein Paar von Antennenleitern genutzt werden. Besonders bevorzugt gelten die genannten Zusammenhänge für die Breite von Antennenleitern beziehungsweise ihre integrierten Kapazitäten jedoch für beliebige Paare von Antennenleitern, die unterschiedliche radiale Abstände von dem Abschirmelement aufweisen. Hierdurch können Abweichungen der Induktivität oder des Wechselstromwiderstandes zwischen verschiedenen Antennenleitern aufgrund einer Abweichung der Verteilung der Antennenleiter oder der Form des Abschirmelements von einer Zylinderform teilweise kompensiert werden.

Die Grundstruktur der Sendeantenne kann der bekannten Struktur von Käfigantennen entsprechen. Antennenleiter können durch Streifenantennen, beispielsweise in Form von Leiterbahnen, oder durch Drähte gebildet werden. Axial endseitig sind jeweils Endringe vorgesehen, über die die verschiedenen Antennenleiter gekoppelt sind. Die einzelnen Endringsegmente, die insbesondere leitend mit den Antennenleitern verbunden sein können, können zu benachbarten Endleitersegmenten leitend oder über eine kapazitive Kopplung, insbesondere über das Abschirmelement, gekoppelt sein. Die Sendeantenne kann näherungsweise weiterhin eine Zylinderform aufweisen, wobei von dieser beispielsweise durch eine leicht ellipsenartige Verformung oder eine Abflachung, also eine sogenannte D-Form, abgewichen werden kann. Die Bezeichnungen axial, also in Richtung der Mittelgerade, radial und in Umfangsrichtung leiten sich aus dieser Grundform ab.

In der bisherigen Diskussion der Variation der Breite der Antennenleiter wurde vernachlässigt, dass eine solche Variation auch zu einer Widerstandsänderung aufgrund eines geänderten Leitungsdurchmessers führen kann. Da Sendeantennen für Magnetresonanzeinrichtungen jedoch mit hohen Sendefrequenzen beschickt werden, kann diese Änderung des Realteils der Impedanz typischerweise verglichen mit der aus der Breitenänderung resultierenden Änderung des Wechselstromwiderstandes, also des Imaginärteils der Impedanz, vernachlässigt werden. Im Rahmen von einer komplexeren Optimierung von Sendeantennen, beispielsweise durch Nutzung von dreidimensionalen Feldsimulationen, kann diese Veränderung des Realteils der Impedanz jedoch selbstverständlich berücksichtigt werden, beispielsweise um eine weitere Verbesserung der Feldhomogenität zu erreichen.

Die Antennenleiter können entlang einer senkrecht auf der Mittelgerade stehenden Ellipse oder einem einseitig abgeflachten Kreis angeordnet sein und/oder ein senkrecht zu der Mittelgerade stehender Querschnitt des Abschirmelements kann ellipsenförmig sein oder die Form eines einseitig abgeflachten Kreises aufweisen. Durch eine Ellipsenform beziehungsweise eine derartige Abflachung kann die Sendeantenne weniger hochbauend realisiert werden, da beispielsweise die Höhe eines auf einer Patientenliege gelagerten Patienten weit geringer ist als die seitliche Ausdehnung einer solchen Patientenliege. Unter der Form eines abgeflachten Kreises ist hierbei insbesondere eine Vergrößerung des Kreisradius in einem Abschnitt des Kreises beziehungsweise eine vollständige Abflachung zu verstehen. Der Abschnitt mit größerem Biegeradius beziehungsweise der abgeflachte Abschnitt kann insbesondere unterhalb einer Patientenliege oder einer anderen Einrichtung zur Lagerung eines Untersuchungsobjekts vorbeigeführt werden. Eine solche Form der Sendeantenne wird auch als D-Form bezeichnet.

Für eine vorgegebene Sendefrequenz kann die Impedanz oder der Imaginärteil der Impedanz aller Antennenleiter im Wesentlichen gleich sein. Hierbei ist unter einer im Wesentlichen gleichen Impedanz eine Impedanz zu verstehen, die zwischen den Antennenleitern um maximal 10 %, vorzugsweise um maximal 5 %, 1 % oder 0,5 %, variiert. Anders ausgedrückt fließen durch die Antennenleiter bei gleicher angelegter Spannung im Wesentlichen die gleichen Antennenströme. Wird die Sendeantenne nun mit zwei Zuleitungen mit einem Sendesignal gleicher Amplitude und mit 90° Phasenversatz beschickt, so kann ein im Wesentlichen homogenes zirkular polarisiertes B1-Feld abgestrahlt werden.

Die Antennenleiter weisen an ihren Enden Endringabschnitte auf, wobei in Umfangsrichtung der Sendeantenne benachbarte Endringabschnitte kapazitiv oder leitend gekoppelt sind, wobei die Ausdehnung der Endringabschnitte der Antennenleiter des Paares in Axialrichtung oder in Umfangsrichtung und Axialrichtung der Sendeantenne unterschiedlich voneinander sind. Die Abmessungen der Endringabschnitte können insbesondere so gewählt werden, dass die aus einem jeweiligen Endringabschnitt und einem radial gegenüberliegenden Abschnitt des Abschirmelements gebildeten Kapazität und/oder die aus der Wechselwirkung dieser Abschnitte resultierende Induktivität für alle Endringabschnitte im Wesentlichen gleich ist. Dies ermöglicht es, nicht nur den Einfluss der Antennenleiter selbst auf den resultierenden Wechselstromwiderstand zu kompensieren, sondern auch den Einfluss der Endringabschnitte, womit die Homogenität des erzeugten B1-Feldes weiter verbessert werden kann.

Neben der erfindungsgemäßen Sendeantenne betrifft die Erfindung eine Magnetresonanzeinrichtung, umfassend eine Sendeantenne und einen Signalgenerator, der zur Bereitstellung wenigstens eines Hochfrequenzsignals an die Sendeantenne eingerichtet ist, wobei die Sendeantenne eine erfindungsgemäße Sendeantenne ist. Vorzugsweise kann der Signalgenerator oder ein zwischen dem Signalgenerator und die Sendeantenne geschalteter Signalteiler zwei Hochfrequenzsignale bereitstellen, die zu separaten Anschlüssen der Sendeantenne geführt sind, wobei die Hochfrequenzsignale einen Phasenversatz von 90° und/oder gleiche Amplituden aufweisen. Es kann somit für die erfindungsgemäße Sendeantenne, bei der der Abstand zwischen den Antennenleitern und dem Abschirmelement variiert, ein einfaches Ansteuerverfahren genutzt werden, wie es auch für Sendeantennen genutzt wird, bei denen der Abstand zwischen Antennenleitern und Abschirmelemente für alle Antennenleitern gleich ist. Durch die Anpassung der Breiten der Antennenleiter beziehungsweise das Variieren der Kapazitäten der Antennenleiter kann vermieden werden, dass eine Inhomogenität des zirkular polarisierten B1-Feldes resultiert beziehungsweise eine solche Inhomogenität kann zumindest weitgehend reduziert werden.

Einerseits können die Antennenleiter entlang einer senkrecht auf der Längsachse stehenden Ellipse oder einem einseitig abgeflachten Kreis angeordnet sein oder andererseits kann ein senkrecht zu der Mittelgerade stehender Querschnitt des Abschirmelements ellipsenförmig sein oder die Form eines einseitig abgeflachten Kreises aufweisen, wobei in beiden Fällen die Hauptachse der Ellipse parallel zu einer Lagerfläche für ein Untersuchungsobjekt, insbesondere zu einer Patientenliege, verläuft oder der jeweilige abgeflachte Abschnitt des Kreises unterhalb der Lagerfläche verläuft.

Durch das beschriebene Vorgehen kann die Bauhöhe der Sendeantenne und somit insbesondere in dem Fall, wenn die Sendeantenne als eine Ganzkörper-Antenne, in der das Untersuchungsobjekt aufgenommen ist, genutzt wird, die gesamte Bauhöhe der Magnetresonanzeinrichtung reduziert werden.

Die Erfindung betrifft zudem ein Verfahren gemäß Anspruch 7 zur Herstellung einer Sendeantenne für eine Magnetresonanzeinrichtung, wobei die Sendeantenne aus mehreren in eine Umfangsrichtung voneinander beabstandet um eine Längsachse herum angeordneten, parallel zu der Längsachse verlaufenden Antennenleitern und einem parallel zu deren Längsachse verlaufenden, die Antennenleiter umgreifenden Abschirmelement aufgebaut wird, wobei wenigstens ein Paar der Antennenleiter derart angeordnet wird, dass der radiale Abstand zwischen einem ersten Antennenleiter des Paars und dem Abschirmelement kleiner ist als der radiale Abstand zwischen einem zweiten Antennenleiter des Paars und dem Abschirmelement, wobei die Breite des ersten Antennenleiters in Umfangsrichtung kleiner als die Breite des zweiten Antennenleiters in Umfangsrichtung gewählt wird und/oder wobei die axialen Enden des ersten Antennenleiters über eine größere Kapazität miteinander gekoppelt werden als die axialen Enden des zweiten Antennenleiters, wobei die Antennenleiter an ihren Enden Endringabschnitte aufweisen, wobei in Umfangsrichtung der Sendeantenne benachbarte Endringabschnitte kapazitiv oder leitend gekoppelt sind, wobei die Ausdehnungen der Endabschnitte der Antennenleiter des Paars in Axialrichtung oder in Umfangsrichtung und Axialrichtung der Sendeantenne unterschiedlich voneinander sind. Das erfindungsgemäße Verfahren kann mit den zu der erfindungsgemäßen Sendeantenne beziehungsweise der erfindungsgemäßen Magnetresonanzeinrichtung erläuterten Merkmalen mit den dort genannten Vorteilen weitergebildet werden und umgekehrt.

Die Breiten der Antennenleiter und/oder die die Enden des jeweiligen Antennenleiters koppelnde Kapazität kann durch Lösung eines Optimierungsproblems zur Optimierung der Feldhomogenität eines zirkular polarisierten B1-Feldes bei einer gegebenen Anregung der Sendeantenne ermittelt werden. Die Optimierung kann iterativ durchgeführt werden, wobei eine vorgegebene Anzahl von Optimierungsschritten durchgeführt werden kann oder die Optimierungsschritte solange wiederholt werden, bis das Verfahren konvergiert, also insbesondere eine vorgegebene maximale Abweichung von einer idealen Homogenität eingehalten wird. Im Rahmen der Optimierung kann insbesondere eine dreidimensionale Feldsimulation genutzt werden.

Weitere Vorteile und Einzelheiten der Erfindung ergeben sich aus den im Folgenden beschriebenen Ausführungsbeispielen sowie den zugehörigen Zeichnungen. Hierbei zeigen schematisch:
- Fig. 1: ein Ausführungsbeispiel einer erfindungsgemäßen Magnetresonanzeinrichtung, die ein Ausführungsbeispiel einer erfindungsgemäßen Sendeantenne umfasst,
- Fig. 2: eine abgewickelte Detaildarstellungen eines Ausführungsbeispiels einer erfindungsgemäßen Sendeantenne,
- Fig. 3: eine abgewickelte Darstellung einer weiteren Sendeantenne, die nicht in den durch die Ansprüche definierten Schutzumfang der Erfindung fällt, und
- Fig. 4: ein weiteres Ausführungsbeispiel einer erfindungsgemäßen Sendeantenne.

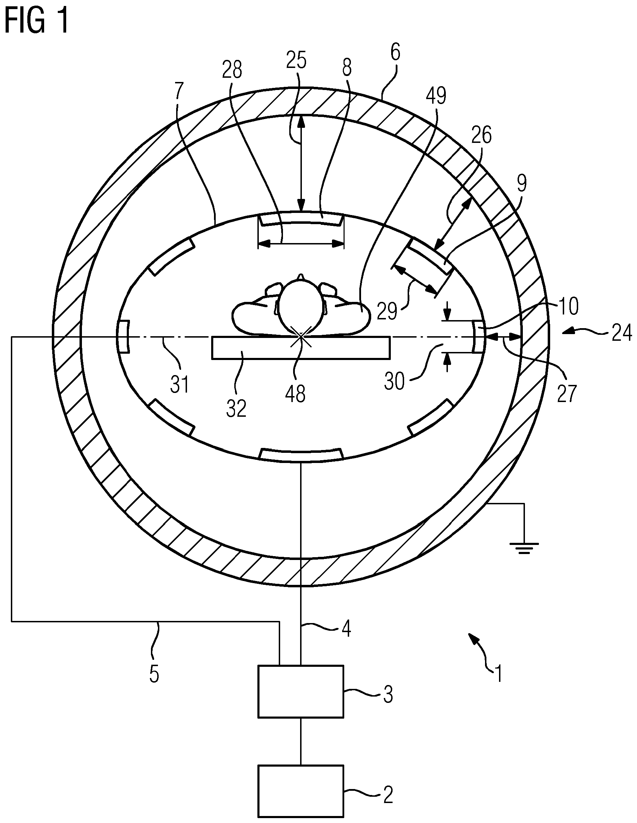

- Fig. 1: zeigt ein Ausführungsbeispiel einer

Magnetresonanzeinrichtung 1, von der aus Übersichtlichkeitsgründen nur die erfindungsrelevanten Komponenten dargestellt sind. Ein Signalgenerator 2 generiert ein Hochfrequenzsignal 4, 5, das über einen Signalteiler 3 der Sendeantenne 24 zugeführt wird, um ein zirkular polarisiertes B1-Feld in ein Untersuchungsvolumen einzustrahlen. Durch dieses kann das Gewebe des Untersuchungsobjekts 49 angeregt werden, um eine Magnetresonanztomographie durchzuführen. Aus Übersichtlichkeitsgründen sind weder Vorrichtungen zur Bereitstellung des Haupt- noch der Gradientenmagnetfelder dargestellt noch Empfangsantennen, Verarbeitungseinrichtungen oder andere Komponenten der Magnetresonanzeinrichtung 1, die nicht unmittelbar der Bereitstellung des B1-Feldes dienen.

Der Signalteiler 3 führt amplitudengleiche jedoch um 90° phasenversetzte Hochfrequenzsignale 4, 5 zu zwei Anschlusspunkten der Sendeantenne 24. Wäre die Sendeantenne 24 zylindersymmetrisch und würde entlang einer Zylinderfläche verteilte, gleiche Antennenleiter aufweisen, so würde hieraus die Abstrahlung eines homogenen, zirkulier polarisierten B1-Feldes resultieren.

Im gezeigten Ausführungsbeispiel sind die Antennenleiter 8 bis 10 jedoch an einem ellipsenförmigen Tragelement 7, beispielsweise an einem Kunststoffträger, befestigt, während das Abschirmelement 6 weiterhin im Wesentlichen zylinderförmig ist. Eine derartige Anordnung kann vorteilhaft sein, da die Lagerfläche 32, im Ausführungsbeispiel eine Patientenliege, und das darauf gelagerte Untersuchungsobjekt 49, nämlich ein Patient, deutlich mehr Platzbedarf in die Querrichtung des Bildes als in die Hochrichtung, aufweisen. Es kann somit zur Reduzierung des Platzbedarfs der Magnetresonanzeinrichtung 1 sowie zur Reduzierung ihrer Kosten vorteilhaft sein, die Antennenleiter 8, die sich senkrecht zur Bildebene und parallel zu der Mittelgerade 48 der Sendeantenne 24 erstrecken, entlang einer Ellipsenform anzuordnen während das Abschirmelement weiterhin kreisförmig ist oder umgekehrt. Die Hauptachse 31 der Ellipse ist hierbei vorzugsweise parallel zu der Lagerfläche 32.

Bei einer solchen Anordnung variiert der radiale Abstand 25 bis 27 der Antennenleiter 8 bis 10 von dem Abschirmelement 6. Das Abschirmelement 6 ist ein Leiter, der auf einem definierten Potential, insbesondere dem Massepotential, liegt. Durch die unterschiedlichen Abstände 25 bis 27 der Antennenleiter 8 bis 10 zu dem Abschirmelement 6 würde bei einer gleichartigen Ausgestaltung der Antennenleiter 8 bis 10 für den Antennenleiter 10 die geringste Induktivität und somit der geringste Wechselstromwiderstand resultieren, während für den Antennenleiter 8 die größte Induktivität und somit der größte Wechselstromwiderstand resultieren würde. Somit würden durch den Antennenleiter 10 erheblich größere Ströme geführt als durch den Antennenleiter 8, wodurch ein inhomogenes B1-Feld sowie hohe Komponentenbelastungen und eine lokal hohe spezifische Absorption durch das Untersuchungsobjekts 49 resultieren können. Um dem entgegen zu wirken, wird für den Antennenleiter 10, der relativ nahe an dem Abschirmelement 6 liegt, eine geringere Breite 30 gewählt als die Breite 28 des Antennenleiters 8, der relativ weit von dem Abschirmelement 6 entfernt ist. für den Antennenleiter 9, der einen mittleren Abstand 26 aufweist, wird eine mittlere Breite 29 gewählt.

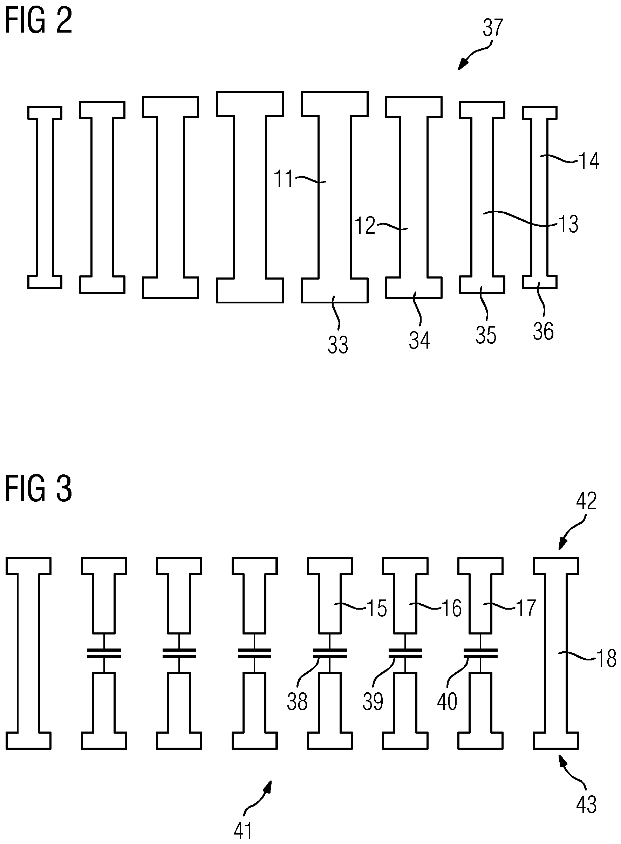

Um dieses Prinzip weiter zu verdeutlichen, ist in Fig. 2 eine abgewickelte Detailansicht einer weiteren Sendeantenne 37 dargestellt, die ausschließlich Antennenleiter 11 bis 14 der Sendeantenne sowie die zur Kopplung der Antennenleiter 11 bis 14 dienenden Ringabschnitte 33 bis 36 zeigt. Die Sendeantenne 37 umfasst sechzehn Antennenleiter 11 bis 14, von denen nur die in der oberen Hälfte der Sendeantenne 37 angeordneten acht Antennenleiter 11 bis 14 dargestellt sind. Die in der Bildmitte gezeigten Antennenleiter 11 sind besonders weit von der nicht gezeigten Abschirmung beabstandet und somit besonders breit ausgebildet. Zur Seite hin fällt für die Antennenleiter 12, 13 und 14 der Abstand zu dem nicht gezeigten Abschirmelement zunehmend ab, womit die Breite der Antennenleiter 12, 13, 14 schrittweise abnimmt. Somit kann erreicht werden, dass die Antennenleiter 11 bis 14 im Wesentlichen die gleiche Induktivität aufweisen, womit sie auch im Wesentlichen einen gleichen Wechselstromwiderstand aufweisen können. Hieraus resultiert eine gleichmäßige Stromverteilung und somit auch ein homogenes B1-Feld.

Die Kopplung der einzelnen Antennenleiter 11 bis 14 erfolgt, wie für entsprechende Sendeantennen aus dem Stand der Technik bekannt ist, über Endringsegmente 33 bis 36, die den einzelnen Antennenleitern 11 bis 14 zugeordnet sind. Die in Fig. 1 dargestellten Zuleitungen für die Hochfrequenzsignale 4, 5 können beispielsweise mit zwei der Endringabschnitte 33 bis 36 verbunden sein. Die verschiedenen Endringabschnitte 33 bis 36 sind jeweils kapazitiv zu dem unmittelbar benachbarten Endringabschnitt 33 bis 36 gekoppelt. Dies kann über zwischengeschaltete Kondensatoren erfolgen, vorzugsweise erfolgt eine Kopplung jedoch darüber, dass beide benachbarten Endringabschnitte 33 bis 36 kapazitiv über die nicht gezeigte Abschirmung gekoppelt sind. Alternativ wäre es auch möglich, die Endringabschnitte 33 bis 36 leitend zu verbinden.

Neben der Induktivität der Antennenleiter 11 bis 14 beeinflusst auch die Gestaltung der Endringabschnitte 33 bis 36 den Stromfluss durch die einzelnen Antennenleiter 11 bis 14. Wie in Fig. 2 dargestellt ist, ist die Ausdehnung der Endringabschnitte in Umfangsrichtung und Axialrichtung der Sendeantenne 37 in Abhängigkeit des Abstands der jeweiligen Antennenleiter 11 bis 14 und ihre Endringabschnitte 33 bis 36 von dem nicht gezeigten Abschirmelement anzupassen.

In einigen Fällen kann die mit Bezug auf Fig. 1 und 2 diskutierte Variation der Breite der Antennenleiter 8 bis 14 nicht gewünscht sein beziehungsweise nicht ausreichen, um einen gleichmäßigen Stromfluss durch die Antennenleiter 8 bis 14 zu erreichen. Ergänzend oder alternativ zur Breitenanpassung kann, wie in Fig. 3 schematisch dargestellt ist, eine zusätzliche Kapazität in den Antennenleitern 15 bis 17 genutzt werden, um den Gesamtwechselstromwiderstand der verschiedenen Antennenleiter 15 bis 18 einander anzugleichen. Die Darstellung und der grundsätzliche Aufbau der in Fig. 3 gezeigten Sendeantenne 41 entspricht der Darstellung und dem Aufbau für die in Fig. 2 gezeigte Sendeantenne 37. Somit ist auch in Fig. 3 der Abstand zwischen dem nicht gezeigten Abschirmelement und den mittig gezeigten Antennenleitern 15 maximal, während der Abstand der Antennenleiter 16, 17, 18 schrittweise abnimmt und für den Antennenleiter 18 sein Minimum erreicht.

Für den am nächsten an der Abschirmung gelegenen Antennenleiter 18 wird im gezeigten Ausführungsbeispiel keine zwischen die axialen Enden 42, 43 des Antennenleiters 18 geschaltete Kapazität genutzt. Der Wechselstromwiderstand des Antennenleiters 18 hängt somit im Wesentlichen ausschließlich von seiner Induktivität ab. Wie vorangehend erläutert würde bei einer gleichen Ausgestaltung der Antennenleiter 15 bis 17 die Induktivität stufenweise mit steigendem Abstand zu der Abschirmung steigen. Um diesen Anstieg der Induktivität zu kompensieren und einen im Wesentlichen gleichen Wechselstromwiderstand für die Antennenleiter 15 bis 18 zu realisieren, werden in den Antennenleitern 15 bis 17 die Kapazitäten 38 bis 40 genutzt, wobei die Kapazität 38 die kleinste Kapazität ist und die Kapazität 40 die größte Kapazität. Prinzipiell könnte im Antennenleiter 18 ebenfalls eine zusätzliche Kapazität genutzt werden, die noch größer ist als die Kapazität 40. Die Nutzung einer solchen Kapazität ist jedoch nicht notwendig, da ein gerader Leiter näherungsweise eine unendliche Kapazität aufweist.

Durch das Vorsehen der Kapazitäten 38 bis 40 wirken die Antennenleiter 15 bis 17 als Reihenschwingkreis. Dies bedeutet, dass bei einer gegebenen Frequenz mit sinkender Kapazität der Betrag des Wechselstromwiderstands der Antennenleiter zunächst sinkt, bis er nach Erreichen der Resonanzbedingung wieder steigt. Werden die Antennenleiter unterresonant betrieben, führt somit die niedrigste Kapazität 38 im Antennenleiter 15 zur stärksten Verringerung des Wechselstromwiderstandes. Dies ermöglicht es, die Kapazitäten 38 bis 40 so zu wählen, dass die aufgrund des zunehmenden Abstands zur Abschirmung steigende Induktivität der Antennenleiter 15 bis 17 durch Vorsehen dieser zusätzlichen Kapazität genau oder zumindest näherungsweise kompensiert wird.

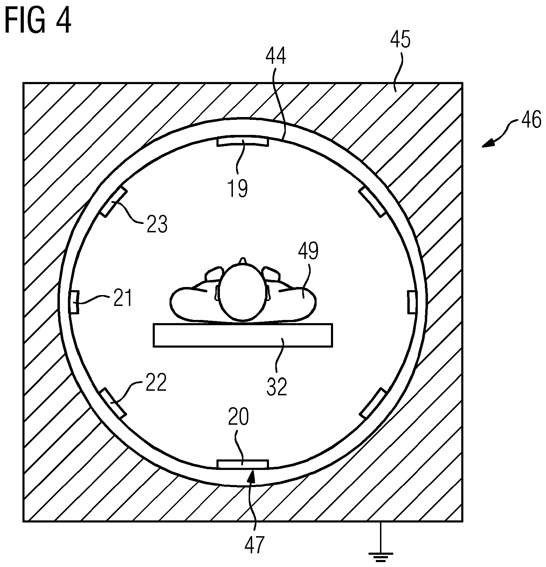

Fig. 4 zeigt ein weiteres Ausführungsbeispiel einer Sendeantenne 46, wobei dieses sich von der in Fig. 1 gezeigten Sendeantenne 24 dadurch unterscheidet, dass ein Tragelement 44, das die Antennenleiter 19 bis 22 trägt, nicht ellipsenförmig ist, sondern die Form eines abgeflachten Kreises beziehungsweise eine D-Form aufweist. Dadurch, dass unterhalb der Lagerfläche 32 ein abgeflachter Abschnitt 47 des kreisförmigen Tragelements 44 mit einem größeren Krümmungsradius vorbeigeführt ist, kann das Tragelement 44 bei gleichem Raumangebot für ein Untersuchungsobjekt 49 insgesamt eine geringere Bauhöhe erreichen.

Wird weiterhin ein im Querschnitt kreisförmiges Abschirmelement 45 genutzt, resultiert auch für diesen Fall eine Variation des radialen Abstandes der Antennenleiter 19 bis 23 von dem Abschirmelement 45. Die Antennenleiter 19 und 20 weisen den maximalen Abstand von dem Abschirmelement 45 auf. Um dies zu kompensieren weisen sie auch die größte Breite auf. Alternativ oder ergänzend könnte, wie vorangehend erläutert, eine relativ kleine Kapazität zwischen die Enden der Antennenleiter 19 und 20 geschaltet werden. Die Antennenleiter 21 und 22 sind nahe an der Wand des Abschirmelements 45 angeordnet und somit relativ schmal ausgebildet beziehungsweise weisen eine relativ große Kapazität oder keine Kapazität auf. Der Antennenleiter 23 weist einen mittleren Abstand und somit auch eine mittlere Breite beziehungsweise mittlere Kapazität auf.

Die vorangehend erläuterten Ansätze können somit auch im Fall einer Anordnung der Antennenleiter 19 bis 23 in einer abgeflachten Kreisform beziehungsweise einer D-Form genutzt werden, um eine gleichmäßige Verteilung des Stroms zwischen den Antennenleitern 19 bis 23 und somit auch eine hohe Feldhomogenität zu erreichen. Simulationen haben ergeben, dass in einer ähnlichen Konfiguration, wie der in Fig. 4 gezeigten Konfiguration, bei einer gleichen Stabbreite die Ströme zwischen den verschiedenen Stäben um mehr als 50 % variieren während die Variation bei optimierter Stabbreite auf ungefähr 10 % reduziert werden kann. Durch das beschriebene Vorgehen kann somit eine erhebliche Verbesserung der Feldhomogenität sowie eine Verringerung der Belastung von Untersuchungsobjekten beziehungsweise von Komponenten der Sendeantenne erreicht werden.

Die gezeigten Beispiele gehen von einer Abweichung der Anordnung der Antennenleiter 8 bis 23 von einer Kreisform bei Beibehaltung einer Kreisform für das Abschirmelement aus. Selbstverständlich ist es auch möglich, dass das Abschirmelement zusätzlich oder alternativ von der Kreisform abweicht. Auch in diesem Fall resultieren unterschiedliche Abstände von dem Abschirmelement für unterschiedliche Antennenleiter. Eine hieraus resultierende Inhomogenität der Stromverteilung bzw. des B1-Felds kann wie obig erläutert kompensiert werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann

hieraus abgeleitet werden, ohne den durch die Ansprüche definierten Schutzumfang der Erfindung zu verlassen.

## Patentansprüche

1. Sendeantenne für eine Magnetresonanzeinrichtung (1) mit mehreren in eine Umfangsrichtung voneinander beabstandet um eine Mittelgerade (48) herum angeordneten, parallel zu der Mittelgerade (48) verlaufenden Antennenleitern (8 - 23) und einem parallel zu der Mittelgerade (48) verlaufenden, die Antennenleiter (8 - 23) umgreifenden Abschirmelement (6, 45), wobei für wenigstens ein Paar der Antennenleiter (8 - 23) der radiale Abstand (25, 26, 27) zwischen einem ersten Antennenleiter (8 - 23) des Paars und dem Abschirmelement (6, 45) kleiner ist als der radiale Abstand (25, 26, 27) zwischen einem zweiten Antennenleiter (8 - 23) des Paars und dem Abschirmelement (6, 45), wobei die Breite des ersten Antennenleiters (8 - 23) in Umfangsrichtung kleiner als die Breite des zweiten Antennenleiters (85 - 23) in Umfangsrichtung ist und/oder wobei die axialen Enden (42, 43) des ersten Antennenleiters (8 - 23) über eine größere Kapazität (38 - 40) miteinander gekoppelt sind als die axialen Enden (42, 42) des zweiten Antennenleiters (8 - 23), wobei die Antennenleiter (8 - 23) an ihren Enden Endringabschnitte (33 - 37) aufweisen, wobei in Umfangsrichtung der Sendeantenne (34, 37, 41, 46) benachbarte Endringabschnitte (33 - 36) kapazitiv oder leitend gekoppelt sind,
**dadurch gekennzeichnet,**
**dass** die Ausdehnungen der Endringabschnitte (33 - 36) der Antennenleiter (8 - 23) des Paares in Axialrichtung der Sendeantenne (34, 37, 41, 46) unterschiedlich voneinander sind.

2. Sendeantenne nach Anspruch 1, **dadurch gekennzeichnet, dass** die Antennenleiter (8 - 23) entlang einer senkrecht auf der Mittelgeraden (48) stehenden Ellipse oder einem einseitig abgeflachten Kreis angeordnet sind und/oder dass ein senkrecht zu der Mittelgerade (48) stehender Querschnitt des Abschirmelements (6, 46) ellipsenförmig ist oder die Form eines einseitig abgeflachten Kreises aufweist.

3. Sendeantenne nach einem der Ansprüche 1 oder 2, **dadurch gekennzeichnet, dass** für eine vorgegebene Sendefrequenz die Impedanz oder der Imaginärteil der Impedanz aller Antennenleiter (8 - 23) im Wesentlichen gleich ist.

4. Magnetresonanzeinrichtung, umfassend eine Sendeantenne (34, 37, 41, 46) und einen Signalgenerator (2), der zur Bereitstellung wenigstens eines Hochfrequenzsignals (4, 5) an die Sendeantenne (24, 37, 41, 46) eingerichtet ist, **dadurch gekennzeichnet, dass** die Sendeantenne (34, 37, 41, 46) eine Sendeantenne (24, 37, 41, 46) nach einem der vorangehenden Ansprüche ist.

5. Magnetresonanzeinrichtung nach Anspruch 4, **dadurch gekennzeichnet, dass** der Signalgenerator (2) oder ein zwischen den Signalgenerator (2) und die Sendeantenne (34, 37, 41, 46) geschalteter Signalteiler (3) dazu eingerichtet ist, zwei Hochfrequenzsignale (4, 5) bereitzustellen, die zu separaten Anschlüssen der Sendeantenne (24, 37, 41, 46) geführt sind, wobei die Hochfrequenzsignale einen Phasenversatz von 90° und/oder gleiche Amplituden aufweisen.

6. Magnetresonanzeinrichtung nach Anspruch 4 oder 5, **dadurch gekennzeichnet, dass** einerseits die Antennenleiter (8 - 23) entlang einer senkrecht auf der Mittelgerade (48) stehenden Ellipse oder einem einseitig abgeflachten Kreis angeordnet sind oder andererseits ein senkrecht zu der Mittelgerade (48) stehender Querschnitt des Abschirmelements (6, 45) ellipsenförmig ist oder die Form eines einseitig abgeflachten Kreises aufweist, wobei in beiden Fällen die Hauptachse (31) der Ellipse parallel zu einer Lagerfläche (32) für ein Untersuchungsobjekt (49), insbesondere zu einer Patientenliege, verläuft oder der jeweilige abgeflachte Abschnitt (47) des Kreises unterhalb der Lagerfläche (32) verläuft.

7. Verfahren zur Herstellung einer Sendeantenne (24, 37, 41, 46) für eine Magnetresonanzeinrichtung (1), wobei die Sendeantenne (34, 37, 41, 46) aus mehreren in eine Umfangsrichtung voneinander beabstandet um eine Mittelgerade (48) herum angeordneten, parallel zu der Mittelgerade (48) verlaufenden Antennenleitern (8 - 23) und einem parallel zu der Mittelgerade (48) verlaufenden, die Antennenleiter (8 - 23) umgreifenden Abschirmelement (6, 45) aufgebaut wird, wobei wenigstens ein Paar der Antennenleiter (8 - 23) derart angeordnet wird, dass der radiale Abstand (25, 26, 27) zwischen einem ersten Antennenleiter (8 - 23) des Paars und dem Abschirmelement (6, 45) kleiner ist als der radiale Abstand (25, 26, 27) zwischen einem zweiten Antennenleiter (8 - 23) des Paars und dem Abschirmelement (6, 45), wobei die Breite (31, 29, 30) des ersten Antennenleiters (8 - 23) in Umfangsrichtung kleiner als die Breite (28, 29, 30) des zweiten Antennenleiters (8 - 23) in Umfangsrichtung gewählt wird und/oder wobei die axialen Enden (42, 43) des ersten Antennenleiters (8 - 23) über eine größere Kapazität (38 - 40) miteinander gekoppelt werden als die axialen Enden (42, 43) des zweiten Antennenleiters (8- 23), wobei die Antennenleiter (8 - 23) an ihren Enden Endringabschnitte (33 - 37) aufweisen, wobei in Umfangsrichtung der Sendeantenne (34, 37, 41, 46) benachbarte Endringabschnitte (33 - 36) kapazitiv oder leitend gekoppelt sind,
**dadurch gekennzeichnet,**
**dass** die Ausdehnungen der Endringabschnitte (33 - 36) der Antennenleiter (8 - 23) des Paares in Axialrichtung der Sendeantenne (34, 37, 41, 46) unterschiedlich voneinander sind.

8. Verfahren nach Anspruch 7, **dadurch gekennzeichnet, dass** die Breiten der Antennenleiter (8 - 23) und/oder die die Enden (42, 43) des jeweiligen Antennenleiters (8 - 23) koppelnde Kapazität (38, 39, 40) durch Lösung eines Optimierungsproblems zur Optimierung der Feldhomogenität eines zirkular polarisierten B1-Feldes bei einer gegebenen Anregung der Sendeantenne (24, 37, 41, 46) ermittelt werden.

9. Verfahren nach Anspruch 8, **dadurch gekennzeichnet, dass** im Rahmen der Optimierung eine dreidimensionale Feldsimulation genutzt wird.

## Claims

1. Transmitting antenna for a magnetic resonance device (1) with a plurality of antenna conductors (8-23) arranged spaced from one another circumferentially around a centre line (48) and extending parallel to the centre line (48) and a screening element (6, 45) extending parallel to the centre line (48) and circumferentially encompassing the antenna conductors (8-23), wherein for at least one pair of the antenna conductors (8-23) the radial distance (25, 26, 27) between a first antenna conductor (8-23) of the pair and the screening element (6, 45) is smaller than the radial distance (25, 26, 27) between a second antenna conductor (8-23) of the pair and the screening element (6, 45), wherein the width of the first antenna conductor (8-23) is smaller in the circumferential direction than the width of the second antenna conductor (85-23) in the circumferential direction and/or wherein the axial ends (42, 43) of the first antenna conductor (8-23) are coupled together by way of a higher capacitance capacitor (38-40) than the axial ends (42, 42) of the second antenna conductor (8-23), wherein the antenna conductors (8-23) have end ring portions (33-37) at their ends, wherein adjacent end ring portions (33-36) in the circumferential direction of the transmitting antenna (34, 37, 41, 46) are coupled capacitively or conductively,
**characterised in that**
the extent of the end ring portions (33-36) of the antenna conductors (8 - 23) of the pair differ from one another in the axial direction of the transmitting antenna (34, 37, 41, 46).

2. Transmitting antenna according to claim 1, **characterised in that** the antenna conductors (8-23) are arranged along an ellipse situated perpendicularly to the centre line (48) or a circle flattened on one side and/or **in that** a cross-section of the screening element (6, 46) situated perpendicularly to the centre line (48) is elliptical or has the shape of a circle flattened on one side.

3. Transmitting antenna according to one of claims 1 or 2, **characterised in that** for a predetermined transmission frequency the impedance or the imaginary part of the impedance of all the antenna conductors (8-23) is substantially identical.

4. Magnetic resonance device, comprising a transmitting antenna (34, 37, 41, 46) and a signal generator (2), which is set up to provide at least one high-frequency signal (4, 5) to the transmitting antenna (24, 37, 41, 46), **characterised in that** the transmitting antenna (34, 37, 41, 46) is a transmitting antenna (24, 37, 41, 46) according to one of the preceding claims.

5. Magnetic resonance device according to claim 4, **characterised in that** the signal generator (2) or a signal splitter (3) connected between the signal generator (2) and the transmitting antenna (34, 37, 41, 46) is set up to provide two high-frequency signals (4, 5), which are guided to separate terminals of the transmitting antenna (24, 37, 41, 46), wherein the high-frequency signals have a phase shift of 90° and/or the same amplitudes.

6. Magnetic resonance device according to claim 4 or 5, **characterised in that** on the one hand the antenna conductors (8-23) are arranged along an ellipse situated perpendicularly to the centre line (48) or a circle flattened on one side or on the other hand a cross-section of the screening element (6, 45) situated perpendicularly to the centre line (48) is elliptical or has the shape of a circle flattened on one side, wherein in both cases the main axis (31) of the ellipse extends parallel to a bearing surface (32) for an object under investigation (49), in particular to a patient couch, or the respective flattened portion (47) of the circle extends underneath the bearing surface (32).

7. Method for producing a transmitting antenna (24, 37, 41, 46) for a magnetic resonance device (1), wherein the transmitting antenna (34, 37, 41, 46) is composed of a plurality of antenna conductors (8-23) arranged spaced from one another circumferentially around a centre line (48) and extending parallel to the centre line (48) and a screening element (6, 45) extending parallel to the centre line (48) and circumferentially encompassing the antenna conductors (8-23), wherein at least one pair of the antenna conductors (8-23) is arranged in such a way that the radial distance (25, 26, 27) between a first antenna conductor (8-23) of the pair and the screening element (6, 45) is smaller than the radial distance (25, 26, 27) between a second antenna conductor (8-23) of the pair and the screening element (6, 45), wherein the width (31, 29, 30) of the first antenna conductor (8-23) is selected to be smaller in the circumferential direction than the width (28, 29, 30) of the second antenna conductor (8-23) in the circumferential direction and/or wherein the axial ends (42, 43) of the first antenna conductor (8-23) are coupled together by way of a higher capacitance capacitor (38-40) than the axial ends (42, 43) of the second antenna conductor (8-23), wherein the antenna conductors (8-23) have end ring portions (33-37) at their ends, wherein adjacent end ring portions (33-36) in the circumferential direction of the transmitting antenna (34, 37, 41, 46) are coupled capacitively or conductively,
**characterised in that**
the extent of the end ring portions (33-36) of the antenna conductors (8 - 23) of the pair differ from one another in the axial direction of the transmitting antenna (34, 37, 41, 46).

8. Method according to claim 7, **characterised in that** the widths of the antenna conductors (8-23) and/or the capacitor (38, 39, 40) coupling the ends (42, 43) of the respective antenna conductor (8-23) are determined by solving an optimisation problem for optimising the field homogeneity of a circularly polarised B1 field in the case of a given excitation of the transmitting antenna (24, 37, 41, 46).

9. Method according to claim 8, **characterised in that** a three-dimensional field simulation is used for the purposes of optimisation.

## Revendications

1. Antenne émettrice d'un dispositif (1) à résonance magnétique comprenant plusieurs conducteurs (8 à 23) d'antenne disposés à distance les uns des autres dans la direction du pourtour autour d'une droite (48) médiane, en s'étendant parallèlement à la droite (48) médiane, et un élément (6, 45) de blindage s'étendant parallèlement à la droite (48) médiane et entourant les conducteurs (8 à 23) d'antenne, dans laquelle, pour au moins une paire des conducteurs (8 à 23) d'antenne, la distance (25, 26, 27) radiale entre un premier conducteur (8 à 23) d'antenne de la paire et l'élément (6, 45) de blindage est plus petite que la distance (25, 26, 27) radiale entre un deuxième conducteur (8 à 23) d'antenne de la paire et l'élément (6, 45) de blindage, dans laquelle la largeur du premier conducteur (8 à 23) d'antenne dans la direction du pourtour est plus petite que la largeur du deuxième conducteur (8 à 23) d'antenne dans la direction du pourtour et/ou dans laquelle les extrémités (42, 43) axiales du premier conducteur (8 à 23) d'antenne sont reliées entre elles par une capacité (38 à 40) plus grande que ne le sont les extrémités (42, 42) axiales du deuxième conducteur (8 à 23) d'antenne, dans laquelle les conducteurs (8 à 23) d'antenne ont à leurs extrémités des parties (33 à 37) annulaires d'extrémité, dans laquelle des parties (33 à 36) annulaires d'extrémité voisines dans la direction du pourtour de l'antenne (34, 37, 41, 46) émettrice sont couplées capacitivement ou conductivement,
**caractérisée**
**en ce que** les étendues des parties (33 à 36) annulaires d'extrémité des conducteurs (8 à 23) d'antenne de la paire dans la direction axiale de l'antenne (34, 37, 41, 46) émettrice sont différentes les unes des autres.

2. Antenne émettrice suivant la revendication 1, **caractérisée en ce que** les conducteurs (8 à 23) d'antenne sont disposés le long d'une ellipse perpendiculaire à la droite (48) médiane ou d'un cercle aplati d'un côté et/ou **en ce qu'**une section transversale perpendiculaire à la droite (48) médiane de l'élément (6, 46) de blindage est en forme d'ellipse ou a la forme d'un cercle aplati d'un côté.

3. Antenne émettrice suivant la revendication 1 ou 2, **caractérisée en ce que**, pour une fréquence d'émission donnée à l'avance, l'impédance ou la partie imaginaire de l'impédance de tous les conducteurs (8 à 23) d'antenne est sensiblement la même.

4. Dispositif à résonance magnétique comprenant une antenne (34, 37, 41, 46) émettrice et un générateur (2) de signal, qui est agencé pour donner au moins un signal (4, 5) en haute fréquence à l'antenne (24, 37, 41, 46) émettrice, **caractérisé en ce que** l'antenne (34, 37, 41, 46) émettrice est une antenne (24, 37, 41, 46) suivant l'une des revendications précédentes.

5. Dispositif à résonance magnétique suivant la revendication 4, **caractérisé en ce que** le générateur (2) de signal ou un diviseur (3) de signal monté entre le générateur (2) de signal et l'antenne (34, 37, 41, 46) émettrice est agencé pour donner deux signaux (4, 5) en haute fréquence qui sont conduits à des bornes distinctes de l'antenne (24, 37, 41, 46) émettrice, dans lequel les signaux en haute fréquence ont un déphasage de 90° et/ou de mêmes amplitudes.

6. Dispositif à résonance magnétique suivant la revendication 4 ou 5, **caractérisé en ce que** d'une part les conducteurs (8 à 23) d'antenne sont disposés le long d'une ellipse perpendiculaire à la droite (48) médiane ou d'un cercle aplati d'un côté ou d'autre part une section transversale perpendiculaire à la droite (48) médiane de l'élément (6, 45) de blindage est en forme d'ellipse ou a la forme d'un cercle aplati d'un côté, dans lequel dans les deux cas l'axe (31) principal de l'ellipse s'étend parallèlement à une surface (32) de pose d'un objet (49) à examiner, en particulier à une couchette de patient, ou la partie (47) aplatie respective du cercle s'étend en dessous de la surface (32) de pose.

7. Procédé de fabrication d'une antenne (24, 37, 41, 46) émettrice d'un dispositif (1) à résonance magnétique, dans lequel on construit l'antenne (34, 37, 41, 46) émettrice à partir de plusieurs conducteurs (8 à 23) d'antenne disposés à distance les uns des autres dans la direction du pourtour autour d'une droite (48) médiane, en s'étendant parallèlement à la droite (48) médiane, et d'un élément (6, 45) de blindage s'étendant parallèlement à la droite (48) médiane et entourant les conducteurs (8 à 23) d'antenne, dans lequel on monte une paire des conducteurs (8 à 23) d'antenne de manière à ce que la distance (25, 26, 27) radiale entre un premier conducteur (8 à 23) d'antenne de la paire et l'élément (6, 45) de blindage soit plus petite que la distance (25, 26, 27) radiale entre un deuxième conducteur (8 à 23) d'antenne de la paire et l'élément (6, 45) de blindage, dans lequel on choisit la largeur (31, 29, 30) du premier conducteur (8 à 23) d'antenne dans la direction du pourtour plus petite que la largeur (28, 29, 30) du deuxième conducteur (8 à 23) d'antenne dans la direction du pourtour et/ou on couple entre elles les extrémités (42, 43) axiales du premier conducteur (8 à 23) d'antenne par une capacité (38, 40) plus grande que ne le sont les extrémités (42, 43) axiales du deuxième conducteur (8 à 23) d'antenne, dans lequel les conducteurs (8 à 23) d'antenne ont à leur extrémité des parties (33 à 37) annulaires d'extrémité, dans lequel les parties (33 à 36) annulaires d'extrémité voisines dans la direction du pourtour de l'antenne (34, 37, 41, 46) émettrice sont couplées capacitivement ou conductivement,
**caractérisé**
**en ce que** les étendues des parties (33 à 36) annulaires d'extrémité des conducteurs (8 à 23) d'antenne de la paire dans la direction axiale de l'antenne (34, 37, 41, 46) émettrice sont différentes les unes des autres.

8. Procédé de fabrication suivant la revendication 7, **caractérisé en ce que** l'on détermine les largeurs des conducteurs (8 à 23) d'antenne et/ou la capacité (38, 39, 40) couplant les extrémités (42, 43) du conducteur (8 à 23) d'antenne respectif en résolvant un problème d'optimisation pour l'optimisation de l'homogénéité d'un champ B1 polarisé circulairement pour une excitation donnée de l'antenne (24, 37, 41, 46) émettrice.

9. Procédé de fabrication suivant la revendication 8, **caractérisé en ce que** dans le cadre de l'optimisation on utilise une simulation de champ en trois dimensions.
